Europäisches Patentamt

European Patent Office

Office européen des brevets

(19)

(11) Publication number: **0 119 848**
A2

# EUROPEAN PATENT APPLICATION

(12)

(21) Application number: 84301797.1

(22) Date of filing: 16.03.84

(51) Int. Cl.³: **A 61 B 17/11**

(30) Priority: 17.03.83 US 476303

(43) Date of publication of application: 26.09.84
Bulletin 84/39

(84) Designated Contracting States: DE GB

(71) Applicant: ETHICON INC., U.S. Route 22, Somerville New
Jersey 08876 (US)

(72) Inventor: Mericle, Robert William, RD 3, Blossom Hill
Road, Lebanon, NJ (US)

(74) Representative: Jones, Alan John et al, CARPMAELS &
RANSFORD 43 Bloomsbury Square, London, WC1A 2RA
(GB)

(54) Anastomotic coupling device.

(57) A three-piece anastomotic coupling device comprising a
pair of cuffs and an interlocking sleeve. The sleeve fits within
the cuff and the entire device fits within the lumen of the
tubular member to be joined.

EP 0 119 848 A2

ANASTOMOTIC COUPLING DEVICE

BACKGROUND OF THE INVENTION

The present invention relates to devices for end-to-end anastomosis of tubular organs and, more particularly, to an anastomotic coupling device for reconnecting the ends of severed gastro-intestinal tract in a manner to promote the healing thereof.

End-to-end anastomosis of the gastric organs may be accomplished by suturing, stapling, or mechanical coupling. Suturing is generally tedious to perform and requires great skill and experience on the part of the surgeon. Suturing is also susceptible to complications resulting from damage to the gastric wall, leakage, potential harboring of infection around the suture material, and the like.

Stapling and mechanical coupling of gastro-intestional structures has been suggested to avoid the disadvantages of suturing and to provide a more reliable fastener and a relatively simple method of anastomosis. Various designs for mechanical coupling devices have been proposed as, for example, in U.S. Patent Nos. 2,453,056, 3,221,746, 3,254,650, 3,774,615, 3,974,835, and 4,214,586.

The ideal anastomotic coupling device should provide perfect adaptation of the tubular members without damage to the gastric wall. The device should provide for quick, sure placement with a minimal possibility for error on the part of the surgeon. It is preferred the device be simple and economical to produce. In many instances, it is desirable that the anastomotic device be completely internal of the gastric lumen. There are other instances where it is desirable that the anastomotic device be substantially contained outside the lumen.

It is accordingly an object of the present invention to provide an anastomotic device particularly adapted to joining gastro-intestional organs. A further object of this invention is to provide a device for joining the ends of interrupted tubular organs of various sizes and functions including, for example, intestines, veins, arteries, lymphatic ducts, ova ducts, and the like. These and other objects of the present invention may be evident from the ensuing description and claims.

## Summary of the Present Invention

The anastomotic coupling device of the present invention consists of three pieces: two tubular cuffs and one locking sleeve. Preferred cuffs consist of a cylinder having an axial bore therein with smooth inner and outer surfaces. It is preferred one end of the cuffs have a shoulder which is used to abut a portion of the sleeve and lock the device in its final configuration in use. The cuffs are sized to fit within the lumen of a tubular member; that is, the tubular member to be joined. The tubular member is inverted over the end of the cuff opposite the end having the shoulder. The sleeve is cylindrical in configuration and has an axial bore therethrough and is sized to fit within the axial bore of the cuffs. The sleeve has means disposed at each end of the sleeve to lock with the cuff in the final device configuration such as an undercut ledge that engages the shoulder at one end of the cuff. In preferred embodiments, the outer surface of the cylindrical member is serrated or has circular grooves on it to aid in holding the tubular member in place.

In use, one end of the tubular member to be joined is passed over the outer surface of a cuff and the tubular member is inverted so that the end fits inside the cuff. The cuff is then placed on one side of the sleeve so that the shoulder abuts against a ledge at the end of the sleeve. The other portion of the tubular member to be joined is placed over the second cuff and inverted so that the end of the tubular member fits inside the cuff. The second cuff with the tubular member inverted thereover is then sliped on the other end of the sleeve and locked in place by an appropriate undercut ledge so that the tubular members abut each other and are locked in place.

The inside diameter of the cuffs is greater than the outside diameter of the sleeve by a sufficient amount to accommodate the wall thickness of the inverted tubular member. The diameters are preferably sized so that a small compressive force is inserted on the wall of the joined tubular member in the assembled connector. As previously mentioned, in the preferred embodiments, the outer wall of the sleeve may contain serrations or otherwise be abraded to aid in holding the tubular member in place. The length of the sleeve and the pair of cuffs are such that when assembled the ends of the cuffs which face each other are spaced apart by a distance slightly less than twice the wall thickness of the tubular member being joined. In this manner the surfaces of the inverted ends of the tubular members are brought into contact under light compression which is effectfve to prevent leakage and is also desirable to promote healing of the joined tissue.

Description of the Drawings

Figure 1 is an exploded view in perspective showing the three pieces of the new anastomotic coupler of the present invention; and

ETH 573

Figure 2 is a cross-sectional view showing the new anastomotic coupler of the present invention joining a tubular vessel together.

## Detailed Description of the Drawings

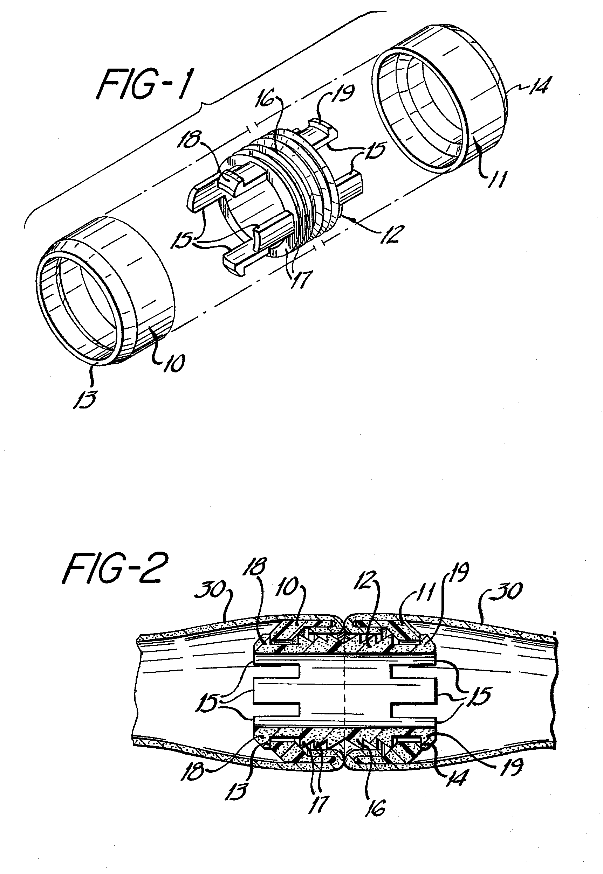

In Figure 1 there is shown a pair of cuffs 10 and 11 and a sleeve 12. The cuffs are identical and are generally cylindrical having a smooth innersurface and a smooth outer surface. In the embodiment depicted there is an offset or shoulder portion 13 and 14 at one end of each cuff. This shoulder portion is formed by tapering the cuff from its outer surface to its inner surface at this end. The sleeve is also cylindrical in nature and the embodiment depicted has four legs 15 depending from each side of central portion 16 of the sleeve. Though in this embodiment the cylinder is discontinuous at each end to form four legs, the cylinder could form any number of legs or in fact be continuous or form only a single leg at each end. The central portion 16 of the sleeve has a plurality of circular grooves 17 or serrations about its periphery. As can be seen more clearly in Figure 2, the tubular member 30 to be joined is passed over the outside surface of a cuff and inverted down into the inside surface of the cuff. The sleeve is then passed down into the inside surface of the cuff so the ledge 18 on at least two of the legs engage the shoulder 13 of the cuff. The other end of the tubular member to be joined has the second cuff 11 inserted within its lumen and the tubular member inverted over the edge of the cuff. The cuff with the tubular member inverted over its end is placed on the opposite end of the sleeve 12 so that the ledge 19 on at least two of the legs at the opposite end of the sleeve engage the shoulder 14 of the cuff 11 to lock the device together and join the tubular members.

The outside diameter of the cuffs should be the same size or slightly smaller than the inside diameter of the tubular member to be joined. The spacing between the cuffs should be slightly less than twice the thickness of the tubular member being joined so that as is seen in Figure 2 slight compression is placed on the tissue in the area where they are abutted. This slight compression establishes hemostatis and aids in the joining and healing of the tissue.

The new coupling devices of the present invention may be manufactured by any convenient technique such as machining or molding. The device may be made from a variety of materials which are known to be bio-compatible in surgical applications. Nylon, polypropylene, and polysulfonate are illustrative of polymeric materials which are readily shaped into the minature pieces of the coupling device. The device may also be fabricated of stainless steel or of biologically absorbable materials such as polylactide, polyglycolide, polydioxanone, copolymers of lactide and glycolide, etc., which are known to hydrolyze in tissue with eventual complete absorption by the body.

The preceding description has been largely directed to a preferred embodiment of the present invention and many variations thereof will be apparent to those skilled in the art. The essential element of the invention is a three-piece coupling device for end-to-end anastomosis of tubular members.

What Is Claimed Is:

1. A three-piece anastomotic coupling device for end-to-end anastomosis of tubular members, said device comprising:

two cuffs and a sleeve, each of said cuffs comprising a cylinder having an axial bore therethrough, said cuff being sized to fit within the lumen of a tubular member with the end of said tubular member inverted over one end of said cuff, said sleeve comprising a cylinder having an axial bore therethrough, said cylinder being sized to fit inside the axial bores of said cuffs with the inverted tubular members in abutting contact outside the cylinder and means for locking said cuffs and sleeve to maintain the inverted ends of the tubular members in contact.

2. A device according to Claim 1 wherein the interlocking means comprises a shoulder on each cuff engaging ledges on opposite ends of the sleeve.

3. A device according to claim 1 or claim 2 wherein the sleeve comprises a cylinder having a central portion and a plurality of legs extending from each end of said central portion.

4. A device accordng to Claim 3 wherein at least two of the legs at each end of the central portion of the sleeve contain undercut ledges, said ledges engaging the ends of the cuffs opposite the ends on which the tubular member is inverted.

5. A device according to any one of claims 1 to 4 wherein the outer surface of the sleeve includes serrations to aid in gripping the tubular member to be joined.

6.    A device according to any one of claims 1 to 5 wherein the inside diameter of the cuff is slightly larger than the outside diameter of the sleeve by a distance sufficient to accommodate the wall thickness of the inverted tubular member.

7.    A device according to any one of claims 1 to 6 wherein the ends of the cuffs facing each other in the assembled device are spaced apart by a distance sufficient to accommodate the combined wall thickness of the inverted tubular members.

8.    A device according to any one of claims 1 to 7 fabricated from a bio-compatible polymeric material.

9.    A device according to Claim 6 wherein said material is absorbable in a biological system.

10.    A three-piece anastomotic coupling device for end-to-end anastomosis of tubular members, said device comprising:

two cuffs and a sleeve, each of said cuffs comprising a cylinder having an axial bore therethrough, said cuff being sized to fit within the lumen of the tubular member, the inner and outer surfaces of the cuffs being smooth, one end of each cuff being tapered towards its inner surface to form a shoulder at said end, said sleeve comprising a generally cylindrical center portion having an axial bore therethrough, a plurality of legs extending axially from each side of said central portion of said sleeve terminating in a radially outward extending ledge adapted to engage the shoulder of a cuff, said sleeve being sized to fit inside the axial bores of said cuffs, whereby when a tubular member to be joined is inverted over the end of one cuff opposite the tapered end and the other tubular member to be joined is inverted over the end of the other cuff opposite its tapered end and that sleeve positioned within the cuffs with the tubular members abutting one another and with the radially outwardly extending ledges on opposite legs of the sleeve engaging the shoulders on said cuffs, the tubular members are joined.

FIG-1

FIG-2